# EUROPÄISCHE PATENTSCHRIFT

(11) **EP 1 080 074 B1**
(45) Veröffentlichungstag und Bekanntmachung des Hinweises auf die Patenterteilung: **08.11.2006**
(21) Anmeldenummer: 99927749.4
(22) Anmeldetag: 25.05.1999
(51) Int. Cl.: C07D 209/14, C07D 209/44, C07D 215/06, C07D 217/04, C07D 401/04, C07D 403/04, A61K 31/40, A61K 31/44, A61K 31/47, A61K 31/495

(54) **Heterocyclische substituierte Amide, deren Herstellung und Verwendung**
Heterocyclic substituted amides, their production and their use
Amides substitués hétérocycliques, leur production et leur utilisation

(30) Priorität: 25.05.1998 DE 19823245
(43) Veröffentlichungstag der Anmeldung: 07.03.2001
(73) Patentinhaber: Abbott GmbH & Co. KG, 65205 Wiesbaden (DE)
(72) Erfinder: LUBISCH, Wilfried, D-69115 Heidelberg (DE); MÖLLER, Achim, D-67269 Grünstadt (DE); TREIBER, Hans-Jörg, D-68782 Brühl (DE); KNOPP, Monika, D-67071 Ludwigshafen (DE)
(74) Vertreter: Grünecker, Kinkeldey, Stockmair & Schwanhäusser Anwaltssozietät
(86) Internationale Anmeldenummer: PCT/EP1999/003549
(87) Internationale Veröffentlichungsnummer: WO 1999/061423

(56) Entgegenhaltungen:
- EP-A- 0 520 336
- WO-A-92/11850
- WO-A-92/12140
- WO-A-94/00095
- WO-A-95/00535
- JP-A- 8 183 771
- S. MEHDI: TRENDS IN BIOLOGICAL SCIENCE, Bd. 16, April 1991 (1991-04), Seiten 150-3, XP002118554 in der Anmeldung erwähnt
- S. L. HARBESON ET AL.: JOURNAL OF MEDICINAL CHEMISTRY, Bd. 37, Nr. 18, 1994, Seiten 2918-29, XP000565669 in der Anmeldung erwähnt
- Z. LI ET AL: JOURNAL OF MEDICINAL CHEMISTRY, Bd. 36, Nr. 22, 1993, Seiten 3472-80, XP000565670 in der Anmeldung erwähnt
- M. R. ANGELASTRO ET AL.: JOURNAL OF MEDICINAL CHEMISTRY, Bd. 33, 1990, Seiten 11-13, XP002058114 in der Anmeldung erwähnt
- J. P. BURKHART ET AL.: TETRAHEDRON LETTERS, Bd. 29, Nr. 28, 1988, Seiten 3433-6, XP002118555 in der Anmeldung erwähnt

## Beschreibung

Die vorliegende Erfindung betrifft neuartige Amide, die Inhibitoren von Enzymen, insbesondere Cystein-Proteasen, wie Calpain (= Calcium dependant cysteine proteases) und dessen Isoenzyme und Cathepsine, zum Beispiel B und L, darstellen.

Calpaine stellen intracelluläre, proteolytische Enzyme aus der Gruppe der sogenannten Cystein-Proteasen dar und werden in vielen Zellen gefunden. Calpaine werden durch erhöhte Calciumkonzentration aktiviert, wobei man zwischen Calpain I oder µ-Calpain, das durch µ-molare Konzentrationen von Calcium-Ionen aktiviert wird, und Calpain II oder m-Calpain, das durch m-molare Konzentrationen von Calcium-Ionen aktiviert wird, unterscheidet (P. Johnson, Int.J.Biochem, **1990**, *22(8)*, 811-22). Heute werden noch weitere Calpain-Isoenzyme postuliert (K.Suzuki et al., Biol.Chem. Hoppe-Seyler, 1995, 376(9),523-9).

Man vermutet, daß Calpaine in verschiedenen physiologischen Prozessen eine wichtige Rolle spielen. Dazu gehören Spaltungen von regulatorischen Proteinen wie Protein-Kinase C, Cytoskelett-Proteine wie MAP 2 und Spektrin, Muskelproteine, Proteinabbau in rheumatoider Arthritis, Proteine bei der Aktivierung von Plättchen, Neuropeptid-Metabolismus, Proteine in der Mitose und weitere, die in. M.J.Barrett et al., Life Sci. 1991., *48*, 1659-69 und K.K.Wang et al., Trends in Pharmacol.Sci., 1994, *15*, 412-9 aufgeführt sind.

Bei verschiedenen pathophysiologischen Prozessen wurden erhöhte Calpain-Spiegel gemessen, zum Beispiel: Ischämien des Herzens (z.B. Herzinfarkt), der Niere oder des Zentralnervensystems (z.B. "Stroke"), Entzündungen, Muskeldystrophien, Katarakten der Augen, Verletzungen des Zentralnervensystems (z.B.Trauma), Alzheimer Krankheit usw. (siehe K.K. Wang, oben). Man vermutet einen Zusammenhang dieser Krankheiten mit erhöhten und anhaltenden intrazellulären Calciumspiegeln. Dadurch werden Calcium-abhängige Prozesse überaktiviert und unterliegen nicht mehr der physiologischen Regelung. Dementsprechend kann eine Überaktivierung von Calpainen auch pathophysiologische Prozesse auslösen.

Daher wurde postuliert, daß Inhibitoren der Calpain-Enzyme für die Behandlung dieser Krankheiten nützlich sein können. Verschiedene Untersuchungen bestätigen dies. So haben Seung-Chyul Hong et al., Stroke 1994, 25(3), 663-9 und R.T.Bartus et al., Neurological Res. 1995, 17, 249-58 eine neuroprotektive Wirkung von Calpain-Inhibitoren in akuten neurodegenerativen Störungen oder Ischämien, wie sie nach Hirnschlag auftreten, gezeigt. Ebenso nach experimentellen Gehirntraumata verbesserten Calpain-Inhibitoren die Erholung der auftretenden Gedächtnisleistungsdefizite und neuromotrischen Störungen (K.E.Saatman et al. Proc.Nazl.Acad.Sci. USA, 1996, 93,3428-3433). C.L.Edelstein et al., Proc.Natl.Acad.Sci. USA, 1995, 92, 7662-6, fand eine protektive Wirkung von Calpain-Inhibitoren auf durch Hypoxie geschädigten Nieren. Yoshida, Ken Ischi et al., Jap.Circ.J. 1995, *59(1),* 40-8, konnten günstige Effekte von Calpain-Inhibitoren nach cardialen Schädigungen aufzeigen, die durch Ischämie oder Reperfusion erzeugt wurden. Da Calpain-Inhibitoren die Freisetzung von dem β-AP4-Protein hemmen, wurde eine potentielle Anwendung als Therapeutikum der Alzheimer Krankheit vorgeschlagen (J.Higaki et al., Neuron, 1995, 14, 651-59). Die Freisetzung von Interleukin-1α wird ebenfalls durch Calpain-Inhibitoren gehemmt (N.Watanabe et al., Cytokine 1994, 6(6), 597-601). Weiterhin wurde gefunden, daß Calpain-Inhibitoren cytotoxische Effekte an Tumorzellen zeigen ( E.Shiba et al. 20th Meeting Int.Ass.Breast Cancer Res., Sendai Jp, 1994, 25.-28.Sept., Int.J.Oncol. *5(Suppl.),* 1994, 381).

Weitere mögliche Anwendungen von Calpain-Inhibitoren sind in K.K.Wang, Trends in Pharmacol.Sci., 1994, 15, 412-8, aufgeführt.

Calpain-Inhibitoren sind in der Literatur bereits beschrieben worden. Überwiegend sind dies jedoch entweder irreversible oder peptidische Inhibitoren, irreversible Inhibitoren sind in der Regel alkylierende Substanzen und haben den Nachteil, daß sie im Organismus unselektiv reagieren oder instabil sind. So zeigen diese Inhibitoren oft unerwünschte Nebeneffekte, wie Toxizität, und sind danach in der Anwendung eingeschränkt oder nicht brauchbar. Zu den irreversiblen Inhibitoren kann man zum Beispiel die Epoxide E 64 (E.B.McGowan et al., Biochem.Biophys.Res.Commun. 1989, *158*, 432-5), α-Halogenketone (H.Angliker et al., J.Med.Chem. 1992, 35, 216-20) oder Disulfide (R.Matsueda et al., Chem.Lett. 1990, 191-194) zählen.

Viele bekannte reversible Inhibitoren von Cystein-Proteasen wie Calpain stellen peptidische Aldehyde dar, insbesondere dipeptidische und tripepidische Aldehyde wie zum Beispiel Z-Val-Phe-H (MDL 28170) (S.Mehdi, Tends in Biol. Sci. 1991, *16*, 150-3). Unter physiologischen Bedingungen haben peptidische Aldehyde den Nachteil, daß sie auf Grund der großen Reaktivität häufig instabil sind, schnell metabolisiert werden können und zu unspezifischen Reaktionen neigen, die die Ursache von toxischen Effekten sein können (J.A.Fehrentz und B.Castro, Synthesis 1983, 676-78.

In JP 08183771 (CA 1996, 605307) und in EP 520336 sind Aldehyde, die sich von Piperidin-4-ylcarbonsäureamiden und 1-Carbonyl-piperidin-4-ylcarbonsäurelamiden ableiten als Calpain-Inhibitoren beschrieben worden. Jedoch sind die hier beanspruchten Aldehyde, die sich von heteroaromatisch substituierten Amiden der allgemeinen Struktur I ableiten bisher noch nicht beschrieben worden.

Peptidische Keton-Derivate sind ebenfalls Inhibitoren von Cystein-Proteasen, insbesondere Calpaine. So sind zum Beispiel bei Serin-Proteasen Keton-Derivate als Inhibitoren bekannt, wobei die Keto-Gruppe von einer elektronenziehenden Gruppe wie CF₃ aktiviert wird. Bei Cystein-Proteasen sind Derivate mit durch CF₃ oder ähnlichen Gruppen aktivierte Ketone wenig oder nicht wirksam (M.R.Angelastro et al., J.Med.Chem. 1990, *33*, 11-13). Überraschenderweise konnten bei Calpain bisher nur Keton-Derivate, bei denen einerseits α-ständige Abgangsgruppen eine irreversible Hemmung verursachen und andererseits ein Carbonsäure-Derivat die Keto-Gruppe aktiviert, als wirksame Inhibitoren gefunden werden (siehe M.R.Angelastro et al., siehe oben; WO 92/11850; WO 92,12140; WO 94/00095 und WO 95/00535). Jedoch sind von diesen Ketoamiden und Ketoestern bisher nur peptidische Derivate als wirksam beschrieben worden (Zhaozhao Li et al., J.Med.Chem. 1993, *36*, 3472-80; S.L.Harbenson et al., J.Med.Chem. 1994, *37*, 2918-29 und siehe oben M.R.Angelastro et al.).

Ketobenzamide sind bereits in der Literatur bekannt. So wurde der Ketoester PhCO-Abu-COOCH₂CH₃ in WO 91/09801, WO 94/00095 und 92/11850 beschrieben. Das analoge Phenyl-Derivat Ph-CONH-CH(CH₂Ph)-CO-COCOOCH₃ wurde in M.R.Angelastro et al., J.Med.Chem. 1990, *33*, 11-13 als jedoch nur schwacher Calpain-Inhibitor gefunden. Dieses Derivat ist auch in J.P.Burkhardt, Tetrahedron Lett., 1988, 3433-36 beschrieben. Die Bedeutung der substituierten Benzamide ist jedoch bisher nie untersucht worden.

In einer Reihe von Therapien wie Schlaganfall werden die Wirkstoffe intravenös zum Beispiel als Infusionslösung appliziert. Dazu ist es notwendig, Substanzen, hier Calpain-Inhibitoren, zur Verfügung zu haben, die ausreichende Wasserlöslichkeit aufweisen, so daß eine Infusionslösung hergestellt werden kann. Viele der beschriebenen Calpain-Inhibitoren haben jedoch den Nachteil, daß sie nur geringe oder keine Wasserlöslichkeit zeigen und somit nicht für eine intravenöse Applikation in Frage kommen. Derartige Wirkstoffe können nur mit Hilfsstoffen, die die Wasserlöslichkeit vermitteln sollen, appliziert werden (vgl. R.T. Bartus et al. *J*. *Cereb. Blood Flow Metab.* 1934, *14,* 537-544). Diese Hilfsstoffe, zum Beispiel Polyethylenalykol, haben aber häufig Begleiteffekte oder sind sogar unverträglich. Ein nicht-peptidischer Calpain-Inhibitor, der also ohne Hilfsstoffe wasserlöslich ist, hätte somit einen großen Vorteil. Ein solcher Inhibitor ist bisher nicht beschrieben worden und wäre damit neu.

In der vorliegenden Erfindung wurden substituierte nicht-peptidische Aldehyde, Ketocarbonsäureester und Ketoamid-Derivate beschrieben. Diese Verbindungen sind neu und zeigen überraschenderweise die Möglichkeit auf, durch Einbau von rigiden strukturellen Fragmenten potente nicht-peptidische Inhibitoren von Cystein-Proteasen, wie z.B. Calpain, zu erhalten. Weiterhin sind bei den vorliegenden Verbindungen der allgemeinen Formel I, die alle mindestens einen aliphatischen Amin-Rest tragen Salz-Bindungen mit Säuren möglich. Eine Vielzahl dieser Substanzen können als 0,5 %ige Lösung Wasserlöslichkeit bei pH = 4-5 aufweisen und damit zeigen sie das gewünschte Profil für eine intravenöse Applikation, wie sie zum Beispiel bei der Schlaganfall-Therapie erforderlich ist.

Gegenstand der vorliegenden Erfindung sind Amide der allgemeinen Formel (I) 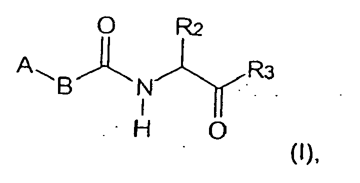 tautomere, enantiomere und diastereomere Formen, sowie physiologisch verträgliche Salze, wobei die Variablen folgende Bedeutung haben:
- kondensierte Cyclen der Formeln 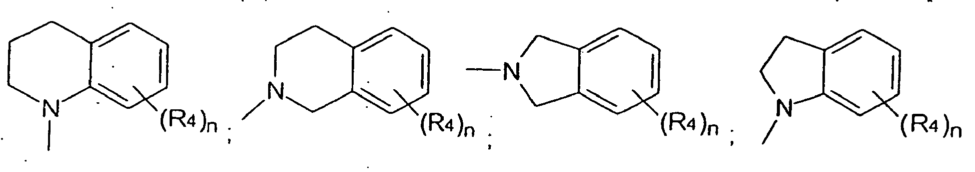 bedeutet,
- B: Pyridyl bedeutet,
- R²: C₁-C₆-Alkyl, verzweigt oder unverzweigt, das einen Phenyl-, Cyclohexyl-, Pyridyl-, Thienyl-, Indolyl- oder Naphthyl-Ring tragen kann, und
- R³: CONH₂ bedeutet,
- R⁴: Wasserstoff oder (CH₂)ₘNR⁸R⁹, O(CH₂)ₘNR⁸R⁹ oder 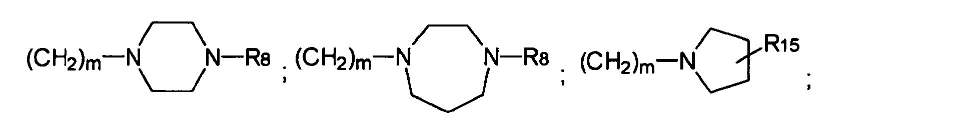 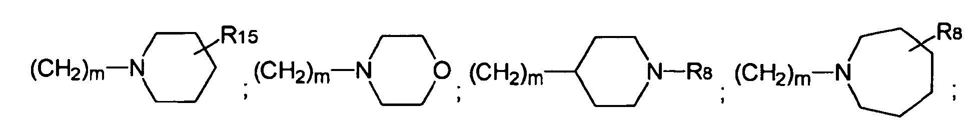 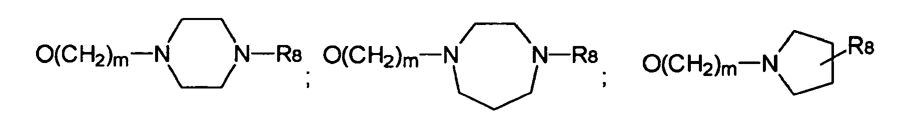 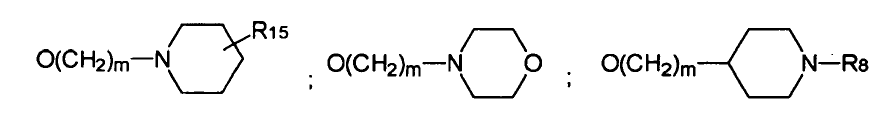 bedeutet und
- R⁸: C₁-C₆-Alkyl, geradlinig oder verzweigt, bedeutet und das mit einem Phenylring substituiert kann, der selbst noch mit einem oder zwei Resten R¹⁰ substituiert sein kann, und
- R⁹: C₁-C₆-Alkyl, geradlinig oder verzweigt, bedeutet und das mit einem Phenylring substituiert kann, der selbst noch mit einem oder zwei Resten R¹⁰ substituiert sein kann, und
- R¹⁰: Wasserstoff, C₁-C₄-Alkyl, verzweigt oder unverzweigt, -O-C₁-C₄-Alkyl, OH, Cl, F, Br, I, CF₃, NO₂, NH₂, CN, CONH₂, COOH, COO-C₁-C₄-Alkyl, -NHCO-C₁-C₄-Alkyl, -NHCO-Phenyl, -NHSO₂-C₁-C₄-Alkyl, -NHSO₂-Phenyl, -SO₂-C₁-C₄-Alkyl und -SO₂₋Phenyl bedeuten kann und
- R¹⁵: Wasserstoff oder die Bedeutung von R⁸,
- m: eine Zahl 1, 2, 3, 4, 5 oder 6 bedeutet und
- n: eine Zahl 0, 1 oder 2 bedeutet.

Die Verbindungen der Formel I können als Racemate, als enantiomerenreine Verbindungen oder als Diastereomere eingesetzt werden. Werden enantiomerereine Verbindungen gewünscht, kann man diese beispielsweise dadurch erhalten, daß man mit einer geeigneten optisch aktiven Base oder Säure eine klassische Racematspaltung mit den Verbindungen der Formel I oder ihren Zwischenprodukten durchführt. Andererseits können die enantiomeren Verbindungen ebenfalls durch Einsatz von kommerziell erwerbbaren Verbindungen, zum Beispiel optisch aktiven Aminosäuren wie Phenylalanin, Tryptophan und Tyrosin, hergestellt werden.

Gegenstand der Erfindung sind auch zu Verbindungen der Formel I mesomere oder tautomere Verbindungen, beispielsweise solche, bei denen die Aldehyd- oder Ketogruppe der Formel I als Enol-Tautomeres vorliegt.

Ein weiterer Gegenstand der Erfindung sind die physiologisch verträglichen Salze der Verbindungen I, die sich durch Umsatz von Verbindungen I mit einer geeigneten Säure oder Base erhalten lassen. Geeignete Säuren und Basen sind zum Beispiel in Fortschritte der Arzneimittelforschung, 1966, Birkhäuser Verlag, Bd.10, S. 224-285, aufgelistet. Dazu zählen zum Beispiel Salzsäure, Citronensäure, Weinsäure, Milchsäure, Phosphorsäure, Methansulfonsäure, Essigsäure, Ameisensäure, Maleinsäure, Fumarsäure usw. bzw. Natriumhydroxid, Lithiumhydroxid und Kaliumhydroxid.

Die Herstellung der erfindungsgemäßen Amide I kann auf verschiedenen Wegen erfolgen, die im Syntheseschema 1 skizziert wurde.

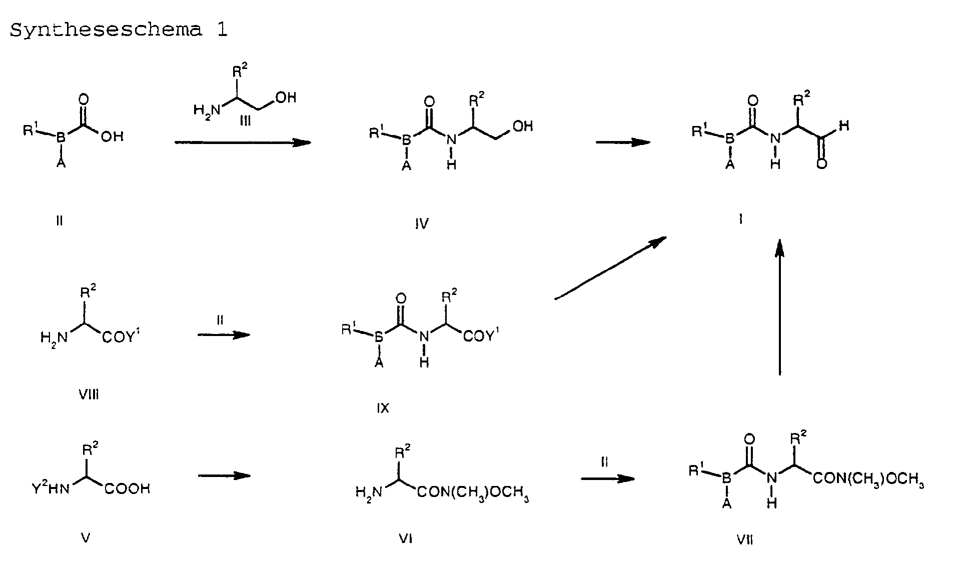

Karbonsäuren II werden mit geeigneten Aminoalkoholen III zu den entsprechenden Amiden IV verknüpft. Dabei benutzt man übliche Peptid-Kupplungs-Methoden, die entweder im C.R.Larock, Comprehensive Organic Transformations, VCH Publisher, 1989, Seite 972f. oder im Houben-Weyl, Methoden der organischen Chemie, 4. Aufl., E5, Kap.V aufgeführt sind. Bevorzugt arbeitet man mit "aktivierten" Säurederivaten von II, wobei die Säureagruppe COOH in eine Gruppe COL überführt wird. L stellt eine Abgangsgruppe wie zum Beispiel Cl, Imidazol und N-Hydroxybenzotriazol dar. Diese aktivierte Säure wird anschließend mit Aminen zu den Amiden IV umgesetzt. Die Reaktion erfolgt in wasserfreien, inerten Lösungsmitteln wie Methylenchlorid, Tetrahydrofuran und Dimethylformamid bei Temperaturen von -20 bis +25°C.

Diese Alkohol-Derivate IV können zu den erfindungsgemäßen Aldehyd-Derivaten I oxidiert werden. Dafür kann man verschiedene übliche Oxidationsreaktionen (siehe C.R.Larock, Comprenhensive Organic Transformations, VCH Publisher, 1989, Seite 604 f.) wie zum Beispiel Swern- und Swern-analoge Oxidationen (T.T.Tidwell, Synthesis 1990, 857-70), Natriumhypochlorid/TEMPO (S.L.Harbenson et al., siehe oben) oder Dess-Martin (J.Org.Chem. 1983, 48, 4155) benutzen. Bevorzugt arbeitet man hier in inerten aprotischen Lösungsmitteln wie Dimethylformamid, Tetrahydrofuran oder Methylenchlorid mit Oxidationsmitteln wie DMSO/py x SO₃ oder DMSO/Oxalylchorid bei Temperaturen von -50 bis +25°C, je nach Methode (siehe obige Literatur).

Alternativ kann man die Karbonsäure II mit Aminohydroxamsäure-Derivate VI zu Benzamiden VII umsetzten. Dabei bedient man sich der gleichen Reaktionsführung wie bei der Darstellung von IV. Die Hydroxam-Derivate VI sind aus den geschützten Aminosäuren V durch Umsatz mit einem Hydroxylamin erhältlich. Dabei benutzt auch hier ein bereits beschriebenes Amidherstellungsverfahren. Die Abspaltung der Schutzgruppe Y², zum Beispiel Boc, erfolgt in üblicherweise, zum Beispiel mit Trifluoressigsäure. Die so erhaltenen Amidhydroxamsäuren VII können durch Reduktion in die erfindungsgemäßen Aldehyde I umgewandelt werden. Dabei benutzt man zum Beispiel Lithiumaluminiumhydrid als Reduktionsmittel bei Temperaturen von -60 bis 0°C in inerten Lösungsmitteln wie Tetrahydrofuran oder Ether.

Analog zum letzten Verfahren kann man auch Karbonsäuren oder Säure-Derivate, wie Ester IX (Y¹ = OR', SR') herstellen, die ebenfalls durch Reduktion in die erfindungsgemäßen Aldehyde I überführt werden können. Diese Verfahren sind in R.C.Larock, Comprehensive Organic Transformations, VCH Publisher, 1989, Seite 619-26 aufgelistet.

Die Herstellung der erfindungsgemäßen heterocyclisch substituierten Amide I, eine Ketoamid- oder Ketoestergruppe tragen, kann auf verschiedenen Wegen erfolgen, die in den Syntheseschemata 2 und 3 skizziert wurden.

Gegebenenfalls werden die Carbonsäureester IIa mit Säuren oder Basen wie Lithiumhydroxid, Natriumhydroxid oder Kaliumhydroxid in wäßrigen Medium oder in Gemischen aus Wasser und organischen Lösungsmitteln wie Alkohole oder Tetrahydrofuran bei Raumtemperatur oder erhöhten Temperaturen, wie 25-100°C, in die Säuren II überführt.

Diese Säuren II werden mit einem α-Aminosäure-Derivat verknüpft, wobei man übliche Bedingungen benutzt, die zum Beispiel im Houben-Weyl, Methoden der organischen Chemie, 4.Aufl., E5, Kap. V, und C.R.Larock, Comprehensive Organic Transformations, VCH Publisher, 1989, *Ch.9* aufgelistet sind.

Zum Beispiel werden die Carbonsäuren II in die "aktivierten" Säure-Derivate IIb =Y-COL überführt, wobei L eine Abgangsgruppe wie Cl, Imidazol und N-Hydroxybenzotriazol darstellt und anschließend durch Zugabe von einem Aminosäure-Derivat H₂N-CH(R²)-COOR in das Derivat XI überführt. Diese Reaktion erfolgt in wasserfreien, inerten Lösungsmitteln wie Methylenchlorid, Tetrahydrofuran und Dimethylformamid bei Temperaturen von -20 bis +25°C.

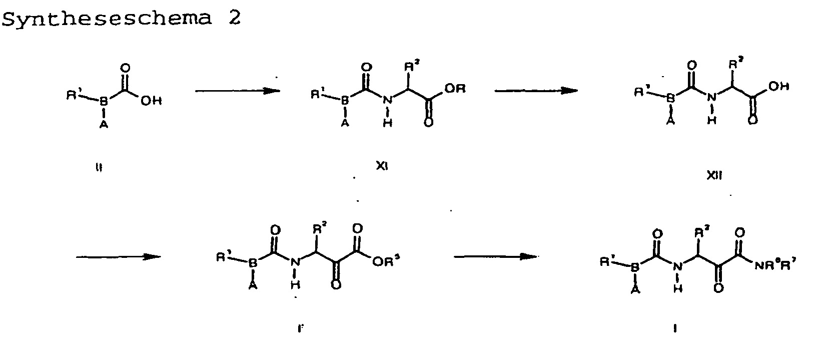

Die Derivate XI, die in der Regel Ester darstellen, werden analog der oben beschriebelnen Hydrolyse in die Ketocarbonsäuren XII überführt. In einer Dakin-West analogen Reaktion werden die Ketoester I' hergestellt, wobei nach einer Methode von ZhaoZhao Li et al.. J.Med.Chem., 1993, *36*, 3472-80 gearbeitet wird. Dabei wird eine Carbonsäure wie XII bei erhöhter Temperatur (50-100°C) in Lösungsmitteln, wie zum Beispiel Tetrahydrofuran, mit Oxalsäuremonoesterchlorid umgesetzt und anschließend das so erhaltene Produkt mit Basen wie Natriumethanolat in Ethanol bei Temperaturen von 25-80°C zum erfindungsgemäßen Ketoester I' umgesetzt. Die Ketoester I' können, wie oben beschrieben, zum Beispiel zu erfindungsgemäßen Ketocarbonsäuren hydrolysiert werden.

Die Umsetzung zu Ketobenzamiden I erfolgt ebenfalls analog der Methode von ZhaoZhao Li et al. (s. oben). Die Ketogruppe in I' wird durch Zugabe von 1,2-Ethandithiol unter Lewissäure-Katalyse, wie zum Beispiel Bortrifluoridetherat, in inerten Lösungsmitteln, wie Methylenchlorid, bei Raumtemperatur geschützt, wobei ein Dithian anfällt. Diese Derivate werden mit Aminen in polaren Lösungsmitteln, wie Alkohole, bei Temperaturen von 0-80°C umgesetzt, wobei die Ketoamide I (R³ = CONR⁶R⁷) anfallen.

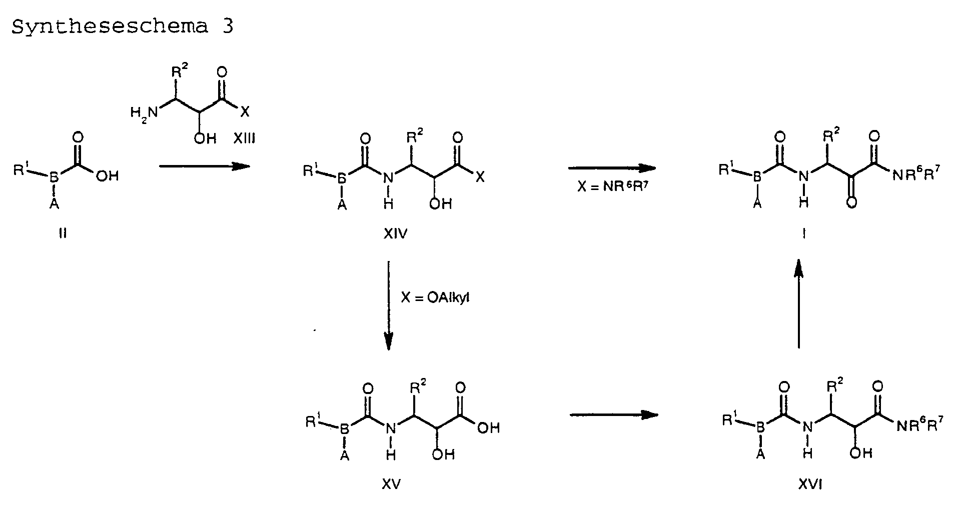

Eine alternative Methode ist im Schema 3 dargestellt. Die Ketocarbonsäuren II werden mit Aminohydroxycarbonsäure-Derivaten XIII (Herstellung von XIII siehe S.L.Harbenson et al., J.Med. Chem. 1994, 37,2918-29 oder J.P. Burkhardt et al. Tetrahedron Lett. 1988, 29, 3433-3436) unter üblichen Peptid-Kupplungs-Methoden (siehe oben, Houben-Weyl) umgesetzt, wobei Amide XIV anfallen. Diese Alkohol-Derivate XIV können zu den erfindungsgemäßen Ketocarbonsäure-Derivaten I oxidiert werden. Dafür kann man verschiedene übliche Oxidationsreaktionen (siehe C.R.Larock, Comprehensive Organic Transformations, VCH Publisher, 1989, Seite 604 f.) wie zum Beispiel Swern- und Swern-analoge Oxidationen, bevorzugt Dimethylsulfoxid/Pyridin-Schwefeltrioxid-Komplex in Lösungsmitteln wie Methylenchlorid oder Tetrahydrofuran, gegebenenfalls unter Zusatz von Dimethylsulfoxid, bei Raumtemperatur oder Temperaturen von -50 bis 25°C, (T.T.Tidwell, Synthesis 1990, 857-70) oder Natriumhypochlorid/TEMPO (S.L.Harbenson et al., siehe oben) benutzen.

Wenn XIV α-Hydroxyester darstellen (X = O-Alkyl), können diese zu Karbonsäuren XV hydrolysiert werden, wobei analog zu den obigen Methoden gearbeitet wird, bevorzugt aber mit Lithiumhydroxid in Wasser/Tetrahydrofuran-Gemischen bei Raumtemperatur. Die Herstellung von anderen Estern oder Amiden XVI erfolgt durch Umsetzung mit Alkoholen oder Aminen unter bereits beschriebenen Kupplungsbedingungen. Das Alkohol-Derivat XVI kann erneut zu erfindungsgemäßen Ketocarbonsäure-Derivaten I oxidiert werden.

Die Herstellung der Carbonsäureester II sind teilweise bereits beschrieben worden oder erfolgt entsprechend üblicher chemischen Methoden.

Die A-B-Bindung wird durch Umsetzung der Halogenaromaten mit den entsprechenden Aminen in Gegenwart von Kaliumcarbonat und 18-Krone-6 in DMF, THF oder BuOH durchgeführt. Die Dialkylaminoalkylsubstituenten werden durch reduktive Aminierung der Aldehydderivate mit den entsprechenden Aminen in Gegenwart von Borhydriden, wie BH₃-Pyridin-Komplex oder oder NaBH₃CN erhalten (A.F. Abdel-Magid, C.A. Maryanoff, K.G. Carson, Tetrahedron Lett. 10990, *31*, 5595; A.E. Moormann, Synth. Commun. 1993, *23*, 789).

Die in der vorliegenden Erfindung enthaltenen heterocyclisch substituierte Amide I stellen Inhibitoren von Cystein-Proteasen dar, insbesondere Cystein-Proteasen wie die Calpaine I und II und Cathepsine B bzw. L.

Die inhibitorische Wirkung der heterocyclisch substituierte Amide I wurde mit in der Literatur üblichen Enzymtests ermittelt, wobei als Wirkmaßstab eine Konzentration des Inhibitors ermittelt wurde, bei der 50 % der Enzymaktivität gehemmt wird (IC₅₀) . Die Amide I wurden in dieser Weise auf Hemmwirkung von Calpain I, Calpain II und Cathepsin 3 gemessen.

### Cathepsin B-Test

Die Cathepsin B-Hemmung wurde analog einer Methode von S.Hasnain et al., J.Biol.Chem. 1993, 268, 235-40 bestimmt. Zu 88 µL Cathepsin B (Cathepsin B aus menschlicher Leber (Calbiochem), verdünnt auf 5 Units in 500 µM Puffer) werden 2 µL einer Inhibitor-Lösung, hergestellt aus Inhibitor und DMSO (Endkonzentrationen: 100 µM bis 0,01 µM). Dieser Ansatz wird für 60 Minuten bei Raumtemperatur (25°C) vorinkubiert und anschließend die Reaktion durch Zugabe von 10 µL 10 mM Z-Arg-Arg-pNA (in Puffer mit 10 % DMSO) gestartet. Die Reaktion wird 30 Minuten bei 405 nM im Kikrotiterplattenreader verfolgt. Aus den maximalen Steigungen werden anschließend die IC₅₀'s bestimmt.

### Calpain I und II Test

Die Testung der inhibitorischen Eigenschaften von Calpain-Inhibitoren erfolgt in Puffer mit 50 mM Tris-HCl, pH 7,5; 0,1 M NaCl; 1 mM Dithiotreithol; 0,11 mM CaCl₂, wobei das fluorogene Calpainsubstrats Suc-Leu-Tyr-AMC (25 mM gelöst in DMSO, Bachem/ Schweiz) verwendet wird. Humanes µ-Calpain wird aus Erythrozyten isoliert und nach mehren chromatographischen Schritten (DEAE-Sepharose, Phenyl-Sepharose, Superdex 200 und Blue-Sepharose) erhält man Enzym mit einer Reinheit >95 %, beurteilt nach SDS-PAGE, Western Blot Analyse und N-terminaler Sequenzierung. Die Fluoreszenz des Spaltproduktes 7-Amino-4-methylcoumarin (AMC) wird in einem Spex-Fluorolog Fluorimeter bei λex = 380 nm und λem = 460 nm verfolgt. In einem Meßbereich von 60 min. ist die Spaltung des Substrats linear und die autokatalytische Aktivität von Calpain gering, wenn die Versuche bei Temperaturen von 12°C durchgeführt werden. Die Inhibitoren und das Calpainsubstrat werden in den Versuchsansatz als DMSO-Lösungen gegeben, wobei DMSO in der Endkonzentration 2 % nicht überschreiten soll.

In einem Versuchsansatz werden 10 µl Substrat (250µM final) und anschließend 10 µl an µ-Calpain (2µg/ml final, d.h. 18 nM) in eine 1 ml Küvette gegeben, die Puffer enthält. Die Calpain-vermittelte Spaltung des Substrats wird für 15-20 min. gemessen. Anschließend Zugabe von 10 µl Inhibitor (50-100 µM Lösung in DMSO) und Messung der Inhibition der Spaltung für weitere 40 min.

Kᵢ-Werte werden nach der klassischen Gleichung für reversible Hemmung bestimmt:
(Methods in Enzymology) Ki = I / (v0/vi) - 1; wobei I = Inhibitorkonzentration, v0 = Anfangsgeschwindiakeit vor Zugabe des Inhibitors; vi = Reaktionsgeschwindigkeit im Gleichgewicht.

Die Geschwindigkeit wird errechnet aus v = Freisetzung AMC/Zeit d.h. Höhe/Zeit.

Calpain ist eine intrazelluläre Cysteinprotease. Calpain-Inhibitoren müssen die Zellmembran passieren, um den Abbau von intrazellulären Proteinen durch Calpain zu verhindern. Einige bekannte Calpain-Inhibitoren, wie zum Beispiel E 64 und Leupeptin, überwinden die Zellmembranen nur schlecht und zeigen dementsprechend, obwohl sie gute Calpain-Inhibitoren darstellen, nur schlechte Wirkung an Zellen. Ziel ist es, Verbindungen mit besser Membrangängigkeit zu finden. Als Nachweis der Membrangängigkeit von Calpain-Inhibitoren benutzen wir humane Plättchen.

### Calpain-vermittelter Abbau der Tyrosinkinase pp60src in Plättchen

Nach der Aktivierung von Plättchen wird die Tyrosinkinase pp60src durch Calpain gespalten. Dies wurde von Oda et al. in J. Biol. Chem., 1993, Vol 268, 12603-12608 eingehend untersucht. Hierbei wurde gezeigt, daß die Spaltung von pp60src durch Calpeptin, einen Inhibitor für Calpain, verhindert werden kann. In Anlehnung an diese Publikation wurde die zellulare Effektivität unserer Substanzen getestet. Frisches humanes, mit Zitrat versetztes Blut wurde 15 min. bei 200g zentrifugiert. Das Plättchen-reiche Plasma wurde gepoolt und mit Plättchenpuffer 1:1 verdünnt (Plättchenpuffer: 68 mM NaCl, 2,7 mM KCl, 0,5 mM MgCl₂ x 6 H₂O, 0,24 mM NaH₂PO₄ x H₂O, 12 mM NaHCO₃, 5,6 mM Glukose, 1 mM EDTA, pH 7,4). Nach einem Zentrifugations- und Waschschritt mit Plättchenpuffer wurden die Plättchen auf 10⁷Zellen/ml eingestellt. Die Isolierung der humanen Plättchen erfolgte bei RT. Im Testansatz wurden isolierte Plättchen (2 x 10⁶) mit unterschiedlichen Konzentrationen an Inhibitoren (gelöst in DMSO) für 5 min. bei 37°C vorinkubiert. Anschließend erfolgte die Aktivierung der Plättchen mit 1 µM Ionophor A23187 und 5 mM CaCl₂. Nach 5 min. Inkubation wurden die Plättchen kurz bei 13000 rpm zentrifugiert und das Pellet in SDS-Probenpuffer aufgenommen (SDS-Probenpuffer: 20 mM Tris-HCl, 5 mM EDTA, 5 mM EGTA, 1 mM DTT, 0,5 mM PMSF, 5 µg/ml Leupeptin, 10 µg/ml Pepstatin, 10 % Glycerin und 1 % SDS). Die Proteine wurden in einem 12 %igen Gel aufgetrennt und pp60src und dessen 52-kDa und 47-kDa Spaltprodukte durch Western-Blotting identifiziert. Der verwendete polyklonale Kaninchen-Antikörper Anti-Cys-src (pp60^{c-src}) wurde von der Firma Biomol Feinchemikalien (Hamburg) erworben. Dieser primäre Antikörper wurde mit einem HRP-gekoppelten zweiten Antikörper aus der Ziege (Boehringer Mannheim, FRG) nachgewiesen. Die Durchführung des Western-Blotting erfolgte nach bekannten Methoden.

Die Quantifizierung der Spaltung von pp60src erfolgte densitometrisch, wobei als Kontrollen nicht-aktivierte (Kontrolle 1: keine Spaltung) und mit Ionophor- und Calcium-behandelte Plättchen (Kontrolle 2: entspricht 100 % Spaltung) verwendet wurden. Der ED₅₀ -Wert entspricht der Konzentration an Inhibitor bei der die Intensität der Farbreaktion um 50 % reduziert wird.

### Glutamat induzierter Zelltod an corticalen Neuronen

Der Test wurde, wie bei Choi D. W., Maulucci-Gedde M. A. and Kriegstein A. R., "Glutamate neurotoxicity in cortical cell culture". *J*. *Neurosci.* 1989, 7, 357-368, durchgeführt.

Aus 15 Tage alten Mäuseembryos wurden die Cortexhälften präpariert und die Einzelzellen enzymatisch (Trypsin) gewonnen. Diese Zellen (Glia und corticale Neuronen) werden in 24 Well-Platten ausgesät. Nach drei Tagen (Laminin beschichteten Platten) oder sieben Tagen (Ornithin beschichteten Platten) wird mit FDU (5-Fluor-2-Desoxyuridine) die Mitosebehandlung durchgeführt. 15 Tage nach der Zellpräparation wird durch Zugabe von Glutamat (15 Minuten) der Zelltod ausgelöst. Nach der Glutamatentfernung werden die Calpaininhibitoren zugegeben. 24 Stunden später wird durch die Bestimmung der Lactatdehydrogenase (LDH) im Zellkulturüberstand die Zellschädigung ermittelt.

Man postuliert, daß Calpain auch eine Rolle im apoptotischen Zelltod spielt (M.K.T.Squier et al. J.Cell.Physiol. 1994, 159, 229-237; T.Patel et al. Faseb Journal 1996, 590, 587-597). Deshalb wurde in einem weiteren Modell in einer humanen Zellinie der Zelltod mit Calcium in Gegenwart eines Calciumionophors ausgelöst. Calpain-Inhibitoren müssen in die Zelle gelangen und dort Calpain hemmen, um den ausgelösten Zelltod zu verhindern.

### Calcium-vermittelter Zelltod in NT2 Zellen

In der humanen Zellinie NT2 läßt sich durch Calcium in Gegenwart des Ionophors A 23187 der Zelltod auslösen. 10⁵ Zellen/well wurden in Mikrotiterplatten 20 Stunden vor dem Versuch ausplattiert. Nach diesem Zeitraum wurden die Zellen mit verschiedenen Konzentrationen an Inhibitoren in Gegenwart von 2,5 µM Ionophor und 5 mM Calcium inkubiert. Dem Reaktionsansatz wurden nach 5 Stunden 0,05 ml XTT (Cell Proliferation Kit II, Boehringer Mannheim) hinzugegeben. Die optische Dichte wird ungefähr 17 Stunden später, entsprechend den Angaben des Herstellers, in dem Easy Reader EAR 400 der Firma SLT bestimmt. Die optische Dichte, bei der die Hälfte der Zellen abgestorben sind, errechnet sich aus den beiden Kontrollen mit Zellen ohne Inhibitoren, die in Abwesenheit und Gegenwart von Ionophor inkubiert wurden.

Bei einer Reihe von neurologischen Krankheiten oder psychischen Störungen treten erhöhte Glutamat-Aktivitäten auf, die zu Zuständen von Übererregungen oder toxischen Effekten im zentralen Nervensystem (ZNS) führen. Glutamat vermittelt seine Effekte über verschiedene Rezeptoren. Zwei von diesen Rezeptoren werden nach den spezifischen Agonisten NMDA-Rezeptor und AMPA-Rezeptor klassifiziert. Antagonisten gegen diese Glutamat vermittelten Effekte können somit zur Behandlung dieser Krankheiten eingesetzt werden, insbesondere zur therapeutischen Anwendung gegen neurodegenerativen Krankheiten wie Chorea Huntington und Parkinsonsche Krankheit, neurotoxischen Störungen nach Hypoxie, Anoxie, Ischämie und nach Lesionen, wie sie nach Schlaganfall und Trauma auftreten, oder auch als Antiepileptika (vgl. Arzneim.Forschung 1990, 40, 511-514; TIPS, 1990, 11, 334-338; Drugs of the Future 1989, 14, 1059-1071).

### Schutz gegen zerebrale Übererregung durch exzitatorische Aminosäuren (NMDA- bzw. AMPA-Antagonismus an der Maus)

Durch intrazerebrale Applikation von exzitatorischen Aminosäuren EAA (Excitatory Amino Acids) wird eine so massive Übererregung induziert, daß diese in kurzer Zeit zu Krämpfen und zum Tod der Tiere(Maus) führt. Durch systemische, z.B. intraperitoneale, Gabe von zentral-wirksamen Wirkstoffen (EAA-Antagonisten) lassen sich diese Symptome hemmen. Da die exzessive Aktivierung von EAA-Rezeptoren des Zentralnervensystems in der Pathogenese verschiedener neurologischer Erkrankungen eine bedeutende Rolle spielt, kann aus dem nachgewiesenen EAA-Antagonismus in vivo auf eine mögliche therapeutische Verwendbarkeit der Substanzen gegen derartige ZNS-Erkrankungen geschlossen werden. Als Maß für die Wirksamkeit der Substanzen wurde ein ED₅₀-Wert bestimmt, bei dem 50 % der Tiere durch eine festgelegte Dosis von entweder NMDA oder AMPA durch die vorangegangene ip.-Gabe der Meßsubstanz syptomfrei werden.

Die heterocyclisch substituierten Amide I stellen Inhibitoren von Cystein-Derivate wie Calpain I bzw. II und Cathepsin B bzw. L dar und können somit zur Bekämpfung von Krankheiten, die mit einer erhöhten Enzymaktivität der Calpain-Enzyme oder Cathepsin-Enzyme verbunden sind, dienen. Die vorliegenden Amide I können danach zur Behandlung von neurodegenerativen Krankheiten, die nach Ischämie, Trauma, Subarachnoidal-Blutungen und Stroke auftreten, und von neurodegenerativen Krankheiten wie multipler Infarkt-Dementia, Alzheimer Krankheit, Huntington Krankheit und von Epilepsien und weiterhin zur Behandlung von Schädigungen des Herzens nach cardialen Ischämien, Schädigungen der Nieren nach renalen Ischämien, Skelettmuskelschädigungen, Muskeldystrophien, Schädigungen, die durch Proliferation der glatten Muskelzellen entstehen, coronaren Vasospasmen, cerebralen Vasospasmen, Katarakten der Augen, Restenosis der Blutbahnen nach Angioplastie dienen. Zudem können die Amide I bei der Chemotherapie von Tumoren und deren Metastasierung nützlich sein und zur Behandlung von Krankheiten, bei denen ein erhöhter Interleukin-1-Spiegel auftritt, wie bei Entzündungen und rheumatischen Erkrankungen, dienen.

Die erfindungsgemäßen Arzneimittelzubereitungen enthalten neben den üblichen Arzneimittelhilfsstoffen eine therapeutisch wirksame Menge der Verbindungen I.

Für die lokale äußere Anwendung, zum Beispiel in Puder, Salben oder Sprays, können die Wirkstoffe in den üblichen Konzentrationen enthalten sein. In der Regel sind die Wirkstoffe in einer Menge von 0,001 bis 1 Gew.-%, vorzugsweise 0,001 bis 0,1 Gew.-% enthalten.

Bei der inneren Anwendung werden die Präparationen in Einzeldosen verabreicht. In einer Einzeldosis werden pro kg Körpergewicht 0,1 bis 100 mg gegeben. Die Zubereitung können täglich in einer oder mehreren Dosierungen je nach Art und Schwere der Erkrankungen verabreicht werden.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes hydriertes Ricinusöl, Polyacrylsäure, Polyethylenglykol, Polyethylenglykostearat, ethoxylierte Fettalkohole, Paraffinöl, Vaseline und Wollfett, verwendet werden. Für die innere Anwendung eignen sich zum Beispiel Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Gleitmittel enthalten sein.

Die neben dem Wirkstoff in der Zubereitung enthaltenen Stoffe sowie die bei der Herstellung der pharmazeutischen Zubereitungen verwendeten Stoffe sind toxikologisch unbedenklich und mit dem jeweiligen Wirkstoff verträglich. Die Herstellung der Arzneimittelzubereitungen erfolgt in üblicher Weise, zum Beispiel durch Vermischung des Wirkstoffes mit anderen üblichen Trägerstoffen und Verdünnungsmitteln.

Die Arzneimittelzubereitungen können in verschiedenen Applikationsweisen verabreicht werden, zum Beispiel peroral, parenteral wie intravenös durch Infusion, subkutan, intraperitoneal und topisch. So sind Zubereitungsformen wie Tabletten, Emulsionen, Infusions- und Injektionslösungen, Pasten, Salben, Gele, Cremes, Lotionen, Puder und Sprays möglich.

### Beispiel 1a

### 2-(1,2,3,4-Tetrahydroisochinolin-2-yl)nikotinsäure-[N-(1-carbamoyl-1-oxo-3-phenylpropan-2-yl)]amid

a) 2-(1,2,3,4-Tetrahydroisochinolin-2-yl)nikotinsäureethylester
   4,0 g (19,4 mmol) 2-Chlornikotinsäureethylester wurden mit 3,2 g (19,4 mmol) 1,2,3,4-Tetrahydroisochinolin-Hydrochlorid und 5,36 g Kaliumcarbonat in 50 ml DMF 3 h unter Rühren auf 110°C erhitzt. Anschließend wurde mit Wasser versetzt, mit Ether extrahiert, die Etherphase mit Ammoniumchlorid gewaschen, getrocknet und eingedampft. Das Rohprodukt wurde chromatographisch (Kieselgel/Heptan-Essigester 20-1) gereinigt, wobei 4,8 g (87 %) anfielen.
b) 2-(1,2,3,4-Tetrahydroisochinolin-2-yl)nikotinsäure
   4,8 g der Zwischenverbindung 1a wurden mit 2N Natronlauge und Ethanol in der Siedehitze verseift (2h). Der Ansatz wurde mit Wasser verdünnt, mit Essigester extrahiert, die Wasserphase sodann mit Essigsäure auf pH 4-5 angesäuert. Durch Absaugen des erhaltenen Niederschlags und nochmaliges Extrahieren der wäßrigen Phase mit Essigester wurden insgesamt 3,3 g (81 %) erhalten.
   Fp. 150-152°C
c) 2-(1,2,3,4-Tetrahydroisochinolin-2-yl)nikotinsäure-[N-(1-carbamoyl-1-ol-3-phenylpropan-2-yl)]amid
   1,65 g (6,5 mmol) der Zwischenverbindung 1c wurden zusammen mit 1,0 ml (7,2 mmol) Triethylamin und 0,88 (6,5 mmol) 1-Hydroxy-1H-benzotriazol (HOBT) bei 0°C in 50 ml DMF vorgelegt und anschließend 1,5 g (6,5 mmol) 3-Amino-2-hydroxy-4-phenylbuttersäureamid-hydrochlorid, 2,7 ml (19,5 mmol) Triethylamin und 1,37 g (7,2 mmol) N'-(3-Dimethylaminopropyl)-N-ethylcarbodiimid-Hydrochlorid (EDC) zugegeben. Nach Rühren über Nacht bei Raumtemperatur wurde mit Wasser und Ether versetzt und der Festkörper abgesaugt, wobei 2,4 g (85 %) anfielen.
   Fp. 237-239°C
d) 2-(1,2,3,4-Tetrahydroisochinolin-2-yl)nikotinsäure-[N-(1-carbamoyl-1-oxo-3-phenylpropan-2-yl)]amid
   1,3 g (3,0 mmol) der Zwischenverbindung 1c wurden mit 1,9 ml (13,6 mmol) Triethylamin bei 0°C in 30 ml DMSO gelöst und anschließend 1,92 g (12 mmol) Pyridin-SO₃-Komplex zugegeben. Nach Rühren über Nacht wurde verd. Natriumhydrogencarbonatlösung zugefügt und mit Essigsäureethylester 3 Mal extrahiert. Die vereinigten Essigesterphasen wurden nach Trocknung eingedampft, der Rückstand mit Methylenchlorid gerührt und der Festkörper anschließend abgesaugt und unter Vakuum getrocknet.
   Ausbeute: 400 mg (31 % d. Th.)
   Fp. 163-165°C
   ¹H-NMR (DMSO-D₆): δ = 2,8-3,2 (6H), 4,3 (2H), 5,4 (1H), 6,8-7,5 (11 H), 7,8-8,1 (3H), 9,0 (1H) ppm.

In analoger Weise wurden die Beispiele 2a, 3a und 5a hergestellt.

### Beispiel 2a

### 2-(1,2,3,4-Tetrahydro-6,7-dimethoxyisochinolin-2-yl)-nikotinsäure-[N-(1-carbamoyl-1-oxo-3-phenylpropan-2-yl)]amid

Fp. 214-216°C
¹H-NMR (DM50-D₆): δ = 2,7-3,5 (6H), 3,8 (6H), 4,3 (2H), 5,5 (1H), 6,7-7,5 (9H), 7,9-8,1 (2H), 9,0 (1H) ppm.

### Beispiel 3a

### 2-(1,2,3,4-Tetrahydro-6,7-dimethoxyisochinolin-2-yl)nikotinsäure-[N-(1-carbamoyl-1-oxohexan-2-yl)]amid

Fp. 182°C
¹H-NMR (DMSO-D₆): δ = 0,9-1,8 (9H), 2, 8 (2H), 3, 5-3, 7 (8H), 4,3 (2H), 5,1 (1H), 6,7-6,9 (3H), 7,7-8,2 (4H), 9,0 (1H) ppm.

### Beispiel 5a

### 4-(1,2,3,4-Tetrahydroisochinolin-2-yl) -nikotinsäure- [N-(1-carbamoyl-1-oxo-3-phenylpropan-2-yl)]amid

Fp. 160-162°C
¹H-NMR (DMSO-D₆): δ = 2,7-3,5 (6H), 4,2-4,5 (2H), 5,5 (1H), 7,0-7,4 (11H), 7,9-8,1 (3H), 9,1 (1H) ppm.

Weitere Beispiele sind der folgenden Tabelle zu entnehmen (Beispiel 1-250).

| | | | | |
|---|---|---|---|---|
| Nr. | (R¹)ₙ-B-CO | A | R² | R³ |
| 3 | 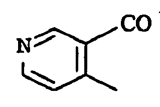 | 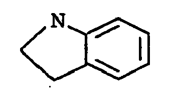 | Bn | CONH₂ |
| 5 | 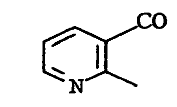 | 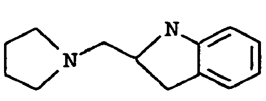 | nBu | CONH₂ |
| 7 | 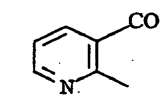 | 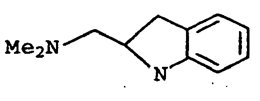 | nBu | CONH₂ |
| 13 | 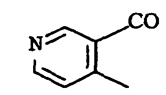 | 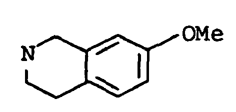 | Bn | CONH₂ |
| 14 | 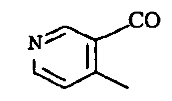 | 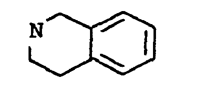 | Bn | CONH₂ |
| 16 | 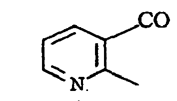 | 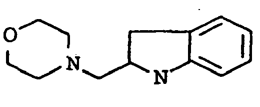 | nBu | CONH₂ |
| 17 | 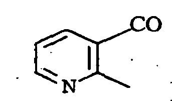 | 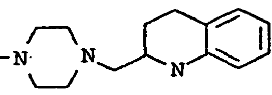 | CH₂cHex | CONH₂ |
| 20 | 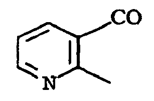 | 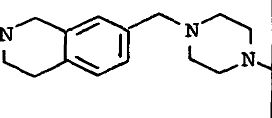 | nBu | CONH₂ |
| 28 | 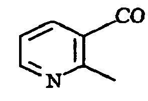 | 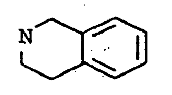 | 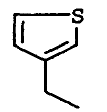 | CONH₂ |
| 29 | 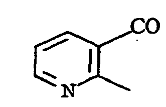 | 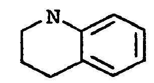 | 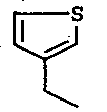 | CONH₂ |
| 31 | 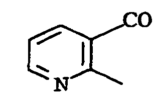 | 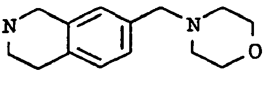 | Bn | CONH₂ |
| 35 | 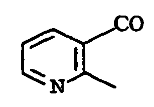 | 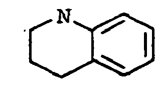 | 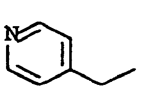 | CONH₂ |
| 38 | 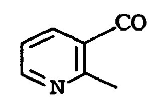 | 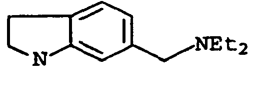 | Bn | CONH₂ |
| 44 | 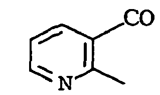 | 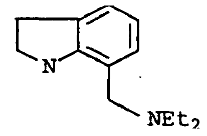 | Bn | CONH₂ |
| 47 | 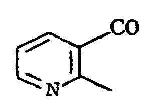 | 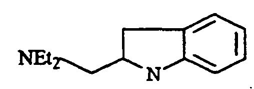 | Bn | CONH₂ |
| 53 | 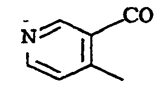 | 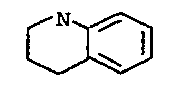 | Bn | CONH₂ |
| 57 | 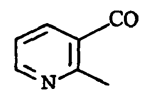 | 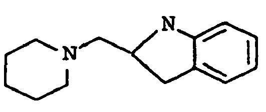 | CH₂cHex | CONH₂ |
| 59 | 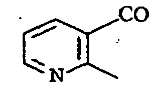 | 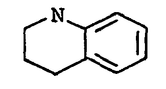 | 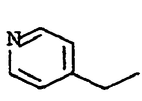 | CONH₂ |
| 62 | 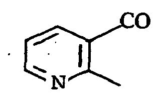 | 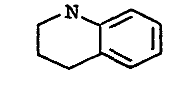 | nBu | CONH₂ |
| 63 | 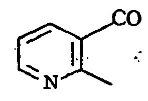 | 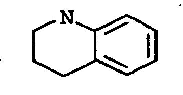 | Bn | CONH₂ |
| 64 | 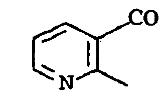 | 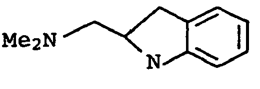 | Bn | CONH₂ |
| 68 | 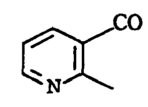 | 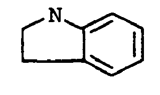 | 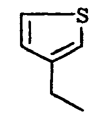 | CONH₂ |
| 69 | 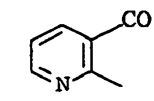 | 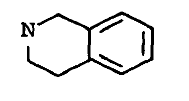 | nBu | CONH₂ |
| 73 | 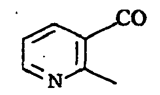 | 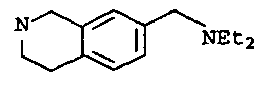 | Bn | CONH₂ |
| 76 | 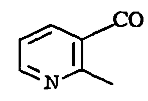 | 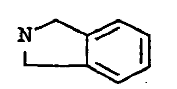 | cHexCH₂ | CONH₂ |
| 80 | 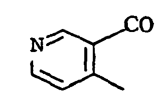 | 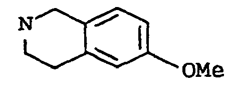 | Bn | CONH₂ |
| 83 | 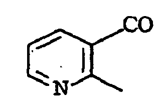 | 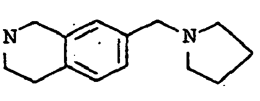 | Bn | CONH₂ |
| 86 | 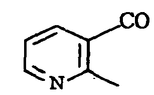 | 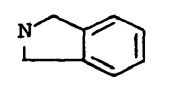 | Bn | CONH₂ |
| 87 | 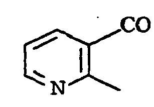 | 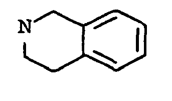 | cHexCH₂ | CONH₂ |
| 88 | 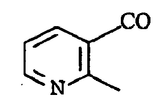 | 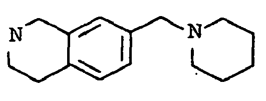 | Bn | CONH₂ |
| 98 | 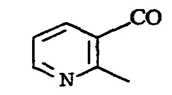 | 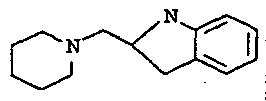 | nBu | CONH₂ |
| 99 | 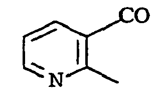 | 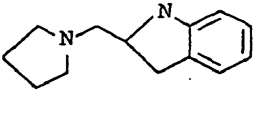 | CH₂cHex | CONH₂ |
| 100 | 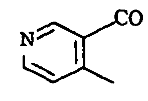 | 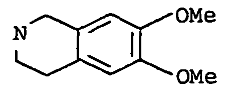 | Bn | CONH₂ |
| 104 | 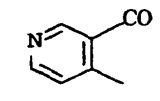 | 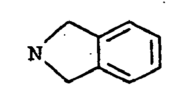 | Bn | CONH₂ |
| 106 | 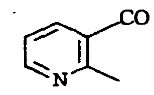 | 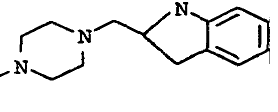 | Bn | CONH₂ |
| 107 | 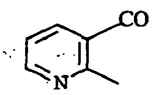 | 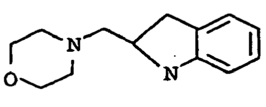 | Bn | CONH₂ |
| 109 | 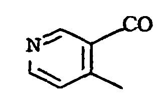 | 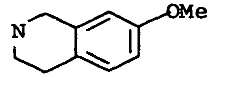 | nBu | CONH₂ |
| 110 | 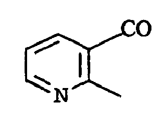 | 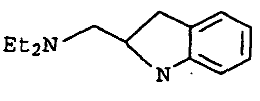 | CH₂cHex | CONH₂ |
| 113 | 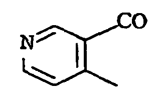 | 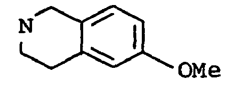 | nBu | CONH₂ |
| 114 | 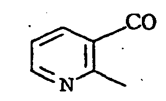 | 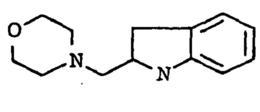 | CH₂cHex | CONH₂ |
| 115 | 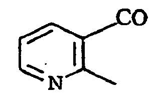 | 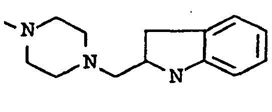 | nBu | CONH₂ |
| 130 | 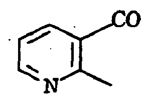 | 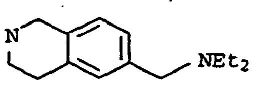 | Bn | CONH₂ |
| 132 | 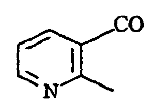 | 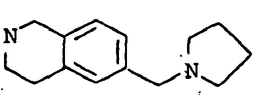 | Bn | CONH₂ |
| 134 | 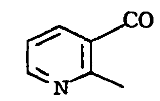 | 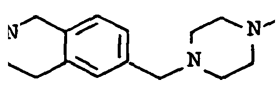 | Bn | CONH₂ |
| 138 | 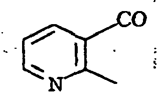 | 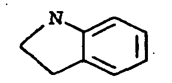 | 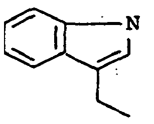 | CONH₂ |
| 146 | 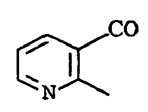 | 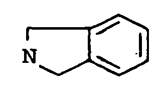 | nBu | CONH₂ |
| 148 | 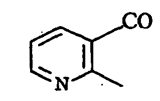 | 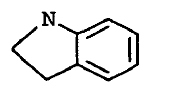 | Bn | CONH₂ |
| 151 | 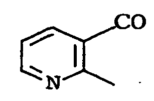 | 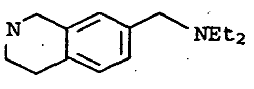 | Bn | CONH₂ |
| 153 | 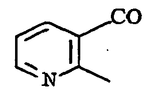 | 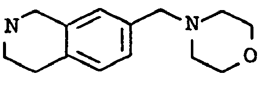 | CH₂cHex | CONH₂ |
| 154 | 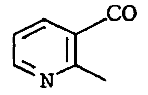 | 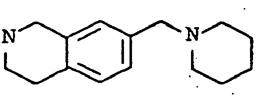 | nBu | CONH₂ |
| 156 | 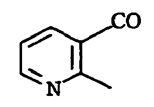 | 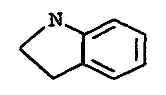 | nBu | CONH₂ |
| 161 | 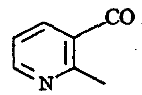 | 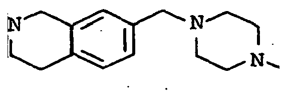 | Bn | CONH₂ |
| 163 | 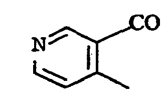 | 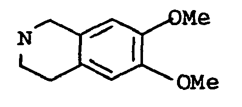 | nBu | CONH₂ |
| 166 | 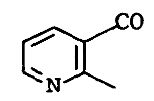 | 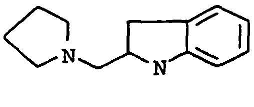 | Bn | CONH₂ |
| 167 | 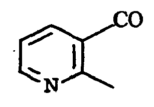 | 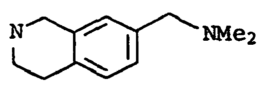 | nBu | CONH₂ |
| 169 | 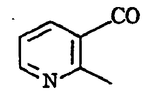 | 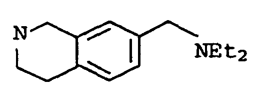 | CH₂cHex | CONH₂ |
| 176 | 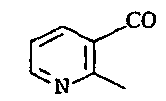 | 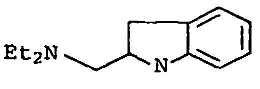 | Bn | CONH₂ |
| 179 | 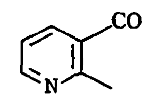 | 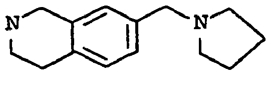 | nBu | CONH₂ |
| 187 | 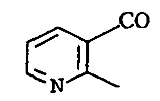 | 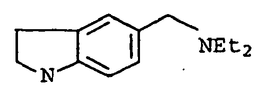 | Bn | CONH₂ |
| 196 | 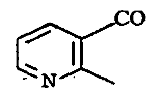 | 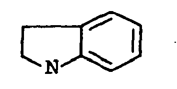 | Bn | CONH₂ |
| 203 | 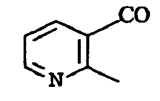 | 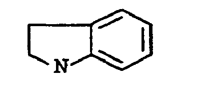 | cHexCH₂ | CONH₂ |
| 204 | 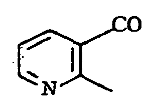 | 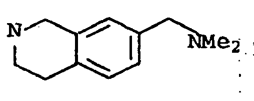 | cHexCH₂ | CONH₂ |
| 206 | 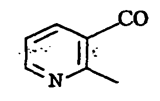 | 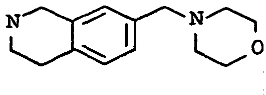 | nBu | CONH₂ |
| 208 | 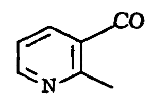 | 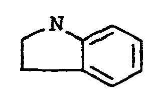 | 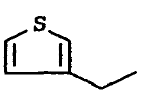 | CONH₂ |
| 213 | 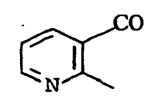 | 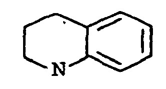 | cHexCH₂ | CONH₂ |
| 215 | 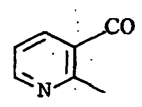 | 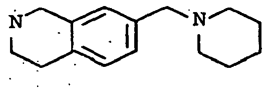 | cHexCH₂ | CONH₂ |
| 216 | 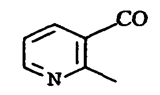 | 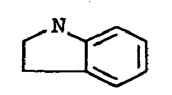 | 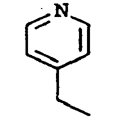 | CONH₂ |
| 218 | 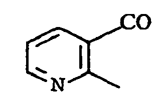 | 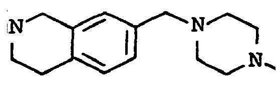 | cHexCH₂ | CONH₂ |
| 221 | 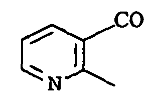 | 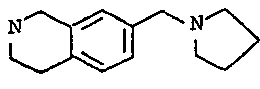 | cHexCH₂ | CONH₂ |
| 222 | 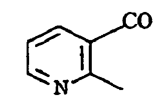 | 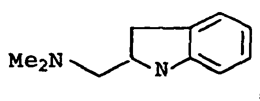 | cHexCH₂ | CONH₂ |
| 228 | 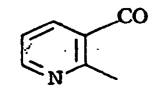 | 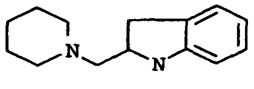 | Bn | CONH₂ |
| 230 | 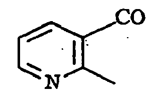 | 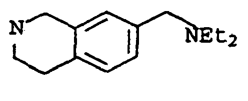 | nBu | CONH₂ |
| 240 | 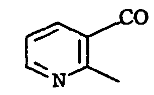 | 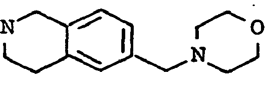 | Bn | CONH₂ |
| 241 | 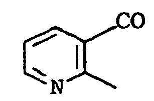 | 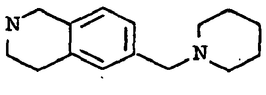 | Bn | CONH₂ |
| 243 | 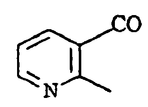 | 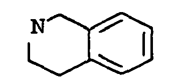 | 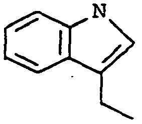 | CONH₂ |
| 246 | 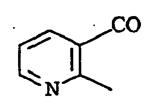 | 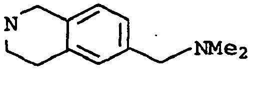 | Bn | CONH₂ |

## Patentansprüche

1. Amide der allgemeinen Formel (I) 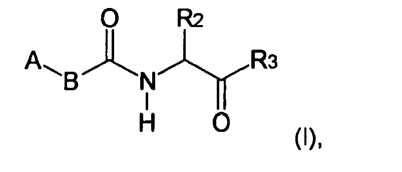 tautomere, enantiomere und diastereomere Formen, sowie physiologisch verträgliche Salze, wobei die Variablen folgende Bedeutung haben:
A kondensierte Cyclen der Formeln 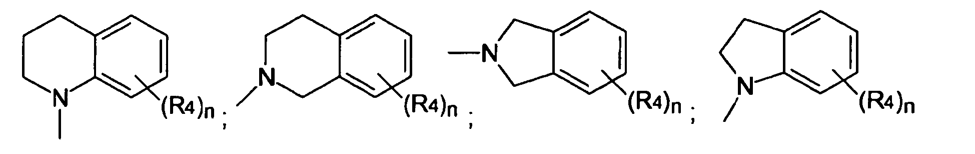 bedeutet,
B Pyridyl bedeutet,
R² C₁-C₆-Alkyl, verzweigt oder unverzweigt, das einen Phenyl-, Cyclohexyl-, Pyridyl-, Thienyl-, Indolyl- oder Naphthyl-Ring tragen kann, und
R³ CONH₂ bedeutet,
R⁴ Wasserstoff oder (CH₂)ₘNR⁸R⁹, O(CH₂)ₘNR⁸R⁹ oder 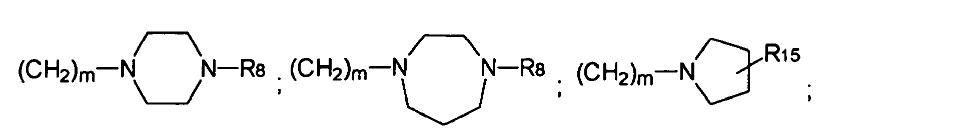 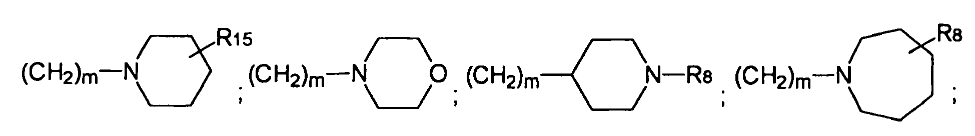 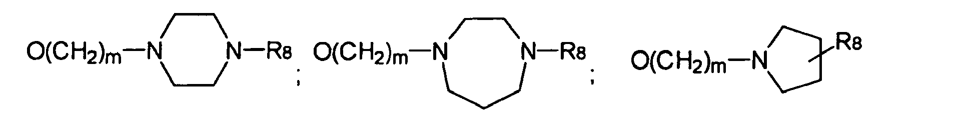 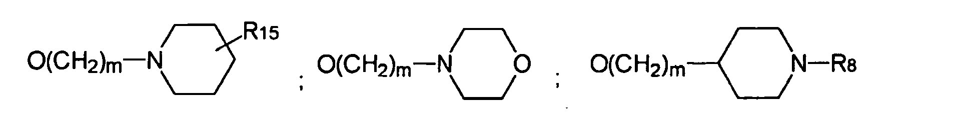 bedeutet und
R⁸ C₁-C₆-Alkyl, geradlinig oder verzweigt, bedeutet und das mit einem Phenylring substituiert kann, der selbst noch mit einem oder zwei Resten R¹⁰ substituiert sein kann, und
R⁹ C₁-C₆-Alkyl, geradlinig oder verzweigt, bedeutet und das mit einem Phenylring substituiert kann, der selbst noch mit einem oder zwei Resten R¹⁰ substituiert sein kann, und
R¹⁰ Wasserstoff, C₁-C₄-Alkyl, verzweigt oder unverzweigt, -O-C₁-C₄-Alkyl, OH, Cl, F, Br, I, CF₃, NO₂, NH₂, CN, CONH₂, COOH, COO-C₁-C₄-Alkyl, -NHCO-C₁-C₄-Alkyl, -NHCO-Phenyl, -NHSO₂-C₁-C₄-Alkyl, -NHSO₂-Phenyl, -SO₂-C₁-C₄-Alkyl und -SO₂-Phenyl bedeuten kann und
R¹⁵ Wasserstoff oder die Bedeutung von R⁸,
m eine Zahl 1, 2, 3, 4, 5 oder 6 bedeutet und
n eine Zahl 0, 1 oder 2 bedeutet.

2. Heterocyclisch substituierte Amide der Formel (I) gemäß Anspruch 1, wobei
A kondensierte Cyclen der Formeln 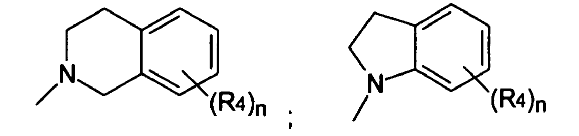 bedeutet.

3. 2-(1,2,3,4-Tetrahydro-6,7-dimethoxyisochino)in-2-yl)-nikotinsäure-[N-(1-carbamoyl-1-oxo-3-phenylpropan-2-yl)]amid.

4. 2-(1,2,3,4-Tetrahydro-6,7-dimethoxyisochinolin-2-yl)-nikotinsäure-[N-(1-carbamoyl-1-oxohexan-2-yl)]amid.

5. Arzneimittel, enthaltend ein Amid nach einem der Ansprüche 1 bis 4.

6. Verwendung eines Amids nach einem der Ansprüche 1 bis 4 zur Herstellung eines Arzneimittels zur Behandlung von Krankheiten, bei denen erhöhte Calpain-Aktivitäten auftreten.

7. Verwendung eines Amids nach einem der Ansprüche 1 bis 4 zur Herstellung eines Arzneimittels zur Behandlung von neuro-degenerativen Krankheiten und neuronalen Schädigungen.

8. Verwendung nach Anspruch 7 zur Behandlung von solchen neuro-degenerativen Krankheiten und neuronalen Schädigungen, die durch Ischämie, Trauma oder Massenblutungen ausgelöst werden.

9. Verwendung nach Anspruch 6 zur Behandlung von Hirnschlag und Schädel-Himtrauma.

10. Verwendung nach Anspruch 6 zur Behandlung der Alzheimerschen Krankheit und der Huntington-Krankheit.

11. Verwendung nach Anspruch 6 zur Behandlung von Epilepsien.

12. Verwendung eines Amids nach einem der Ansprüche 1 bis 4 zur Herstellung eines Arzneimittels zur Behandlung von Schädigungen des Herzens nach cardialen Ischämien, Schädigungen der Nieren nach renalen Ischämien, Skelettmuskelschädigungen, Muskeldystrophien, Schädigungen, die durch Proliferation der glatten Muskelzellen entstehen, coronarer Vasospasmus, cerebraler Vasospasmus, Katarakten der Augen und Restenosis der Blutbahnen nach Angioplastie.

13. Verwendung eines Amids nach einem der Ansprüche 1 bis 4 zur Herstellung eines Arzneimittels zur Behandlung von Tumoren und deren Metastasierung.

14. Verwendung eines Amids nach einem der Ansprüche 1 bis 4 zur Herstellung eines Arzneimittels zur Behandlung von Krankheiten, bei denen erhöhte Interleukin-1-Spiegel auftreten.

15. Verwendung eines Amids nach einem der Ansprüche 1 bis 4 zur Herstellung eines Arzneimittels zur Behandlung von immunologischen Krankheiten wie Entzündungen und rheumatischen Erkrankungen.

16. Arzneimittelzubereitungen zur peroralen, parenteralen und intraperitonalen Anwendung, enthaltend pro Einzeldosis, neben den üblichen Arzneimittelhilfsstoffen, mindestens ein Amid nach einem der Ansprüche 1 bis 4.

## Claims

1. Amides of the general formula (I) 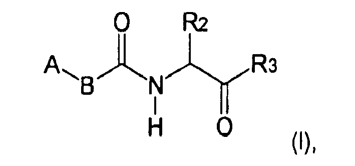 tautomeric, enantiomeric and diastereomeric forms, and physiologically acceptable salts, the variables having the following meaning:
A means fused cycles of the formulae 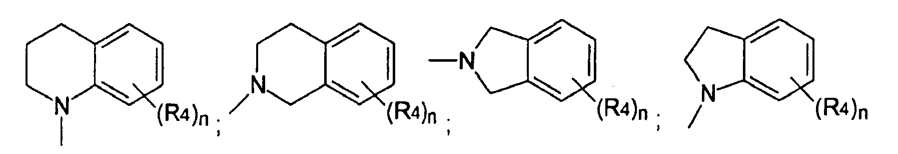
B means pyridyl,
R² means C₁-C₆ alkyl, branched or unbranched, which may bear a phenyl, cyclohexyl, pyridyl, thienyl, indolyl or naphthyl ring, and
R³ means CONH₂,
R⁴ means hydrogen or (CH₂)ₘNR⁸R⁹, O(CH₂)ₘNR⁸R⁹ or 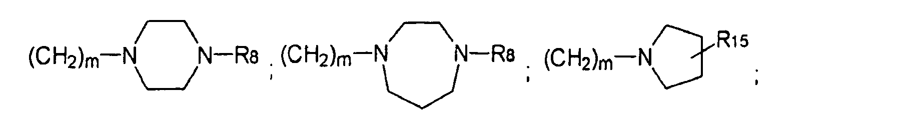 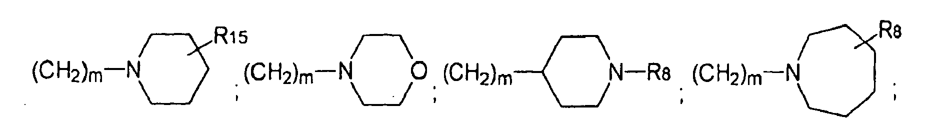 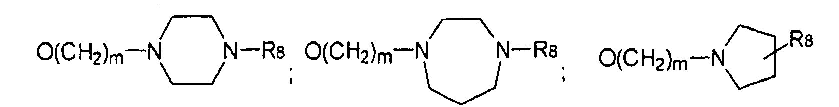 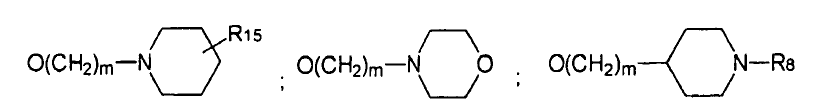 and
R⁸ means C₁-C₆ alkyl, linear or branched, and which may be substituted with a phenyl residue, which may itself additionally be substituted with one or two residues R¹⁰, and
R⁹ means C₁-C₆ alkyl, linear or branched, and which may be substituted with a phenyl residue, which may itself additionally be substituted with one or two residues R¹⁰, and
R¹⁰ may mean hydrogen, C₁-C₄ alkyl, branched or unbranched, -O-C₁-C₄-alkyl, OH, Cl, F, Br, I, CF₃, NO₂, NH₂, CN, CONH₂, COOH, COO-C₁-C₄-alkyl, -NHCO-C₁-C₄-alkyl, -NHCO-phenyl, -NHSO₂-C₁-C₄-alkyl, -NHSO₂-phenyl, -SO₂-C₁-C₄-alkyl and -SO₂-phenyl and
R¹⁵ means hydrogen or has the meaning of R⁸,
m means a number 1, 2, 3, 4, 5 or 6 and
n means a number 0, 1 or 2.

2. Heterocyclic substituted amides of the formula (I) according to claim 1, wherein
A means fused cycles of the formulae

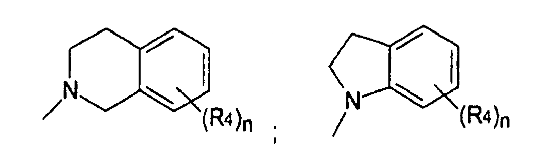

3. 2-(1,2,3,4-Tetrahydro-6,7-dimethoxyisoquinolin-2-yl)-nicotinic acid [N-(1-carbamoyl-1-oxo-3-phenylpropan-2-yl)]amide.

4. 2-(1,2,3,4-Tetrahydro-6,7-dimethoxyisoquinolin-2-yl)-nicotinic acid [N-(1-carbamoyl-1-oxohexan-2-yl)]amide.

5. A pharmaceutical preparation containing an amide according to any one of claims 1 to 4.

6. Use of an amide according to any one of claims 1 to 4 for the production of a pharmaceutical preparation for the treatment of diseases in which increased levels of calpain activity occur.

7. Use of an amide according to any one of claims 1 to 4 for the production of a pharmaceutical preparation for the treatment of neurodegenerative diseases and neuronal damage.

8. Use according to claim 7 for the treatment of neurodegenerative diseases and neuronal damage which are initiated by ischaemia, trauma or haemorrhage.

9. Use according to claim 6 for the treatment of stroke and craniocerebral trauma.

10. Use according to claim 6 for the treatment of Alzheimer's disease and Huntington's disease.

11. Use according to claim 6 for the treatment of epilepsy.

12. Use of an amide according to any one of claims 1 to 4 for the production of a pharmaceutical preparation for the treatment of heart damage after cardiac ischaemia, kidney damage after renal ischaemia, skeletal muscle damage, muscular dystrophies, damage arising from the proliferation of smooth muscle cells, coronary vasospasm, cerebral vasospasm, cataracts of the eye and restenosis of blood vessels after angioplasty.

13. Use of an amide according to any one of claims 1 to 4 for the production of a pharmaceutical preparation for the treatment of tumours and the metastasisation thereof.

14. Use of an amide according to any one of claims 1 to 4 for the production of a pharmaceutical preparation for the treatment of diseases in which increased interleukin-1 levels occur.

15. Use of an amide according to any one of claims 1 to 4 for the production of a pharmaceutical preparation for the treatment of immunological diseases such as inflammatory and rheumatic diseases.

16. Pharmaceutical dosage forms for oral, parenteral and intraperitoneal administration, each individual dose containing, apart from conventional pharmaceutical excipients, at least one amide according to any one of claims 1 to 4.

## Revendications

1. Amide de la formule générale (I) : 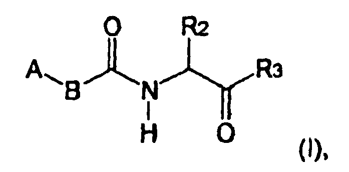 ses formes tautomères, énantiomères et diastéréomères, ainsi que les sels physiologiquement compatibles, où les variables ont la signification suivante :
A représente des cycles condensés des formules : 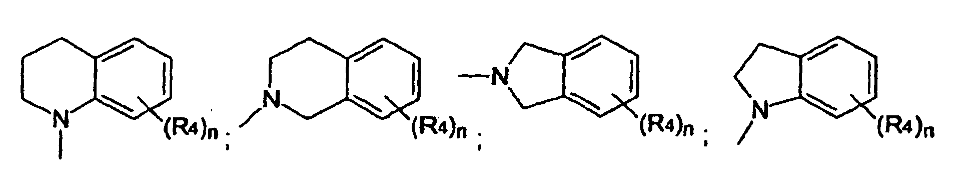
B représente pyridyle,
R² représente un alkyle en C₁-C₆, ramifié ou non ramifié, qui peut porter un cycle phényle, cyclohexyle, pyridyle, thiényle, indolyle ou naphtyle, et
R³ représente CONH₂,
R⁴ représente hydrogène ou (CH₂)ₘNR⁸R⁹, O(CH₂)ₘNR⁸R⁹, ou 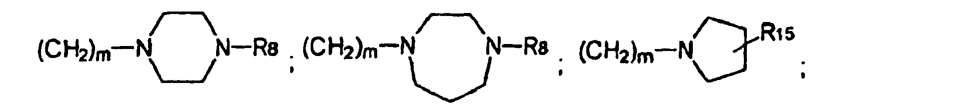 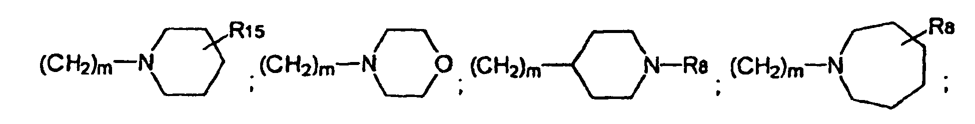 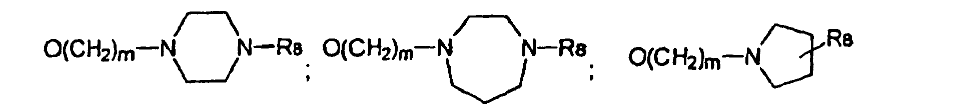 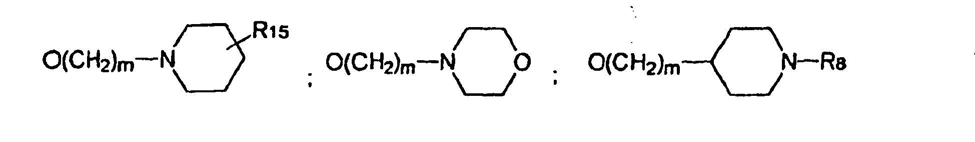 et
R⁸ représente un alkyle en C₁-C₆, linéaire ou ramifié et qui peut être substitué avec un cycle phényle, qui peut être lui-même substitué par un ou deux restes R¹⁰, et
R⁹ représente un alkyle en C₁-C₆, linéaire ou ramifié et qui peut être substitué avec un cycle phényle, qui peut être lui-même substitué par un ou deux restes R¹⁰, et
R¹⁰ représente hydrogène, un alkyle en C₁-C₄, ramifié ou non ramifié, -O-alkyle en C₁₋C₄, OH, CI, F, Br, I, CF₃, NO₂, NH₂, CN, CONH₂, COOH, -COO-alkyle en C₁-C₄, -NHCO-alkyle en C₁-C₄, -NHCO-phényle, -NHSO₂-alkyle en C₁-C₄, -NHSO₂-phényle, -SO₂-alkyle en C₁-C₄, et -SO₂-phényle,
R¹⁵ représente hydrogène ou a la signification de R⁸,
m représente le chiffre 1, 2, 3, 4, 5 ou 6, et
n représente le chiffre 0, 1 ou 2.

2. Amide substitué hétérocyclique de la formule (I) selon la revendication 1, où A représente des cycles condensés des formules :

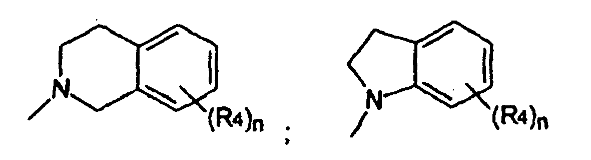

3. [N-(1-carbamoyl-1-oxo-3-phénylpropan-2-yl)]-amide de l'acide 2-(1,2,3,4-tétrahydro-6,7-diméthoxy-isoquinoléin-2-yl)nicotinique.

4. [N-(1-carbamoyl-1-oxohexan-2-yl)]amide de l'acide 2-(1,2,3,4-tétrahydro-6,7-diméthoxy-isoquinoléin-2-yl)nicotinique.

5. Agent pharmaceutique contenant un amide selon l'une des revendications 1 à 4.

6. Utilisation d'un amide selon l'une des revendications 1 à 4, pour la préparation d'un agent pharmaceutique pour le traitement de maladies, dans lesquelles des activités élevées de la calpaïne se présentent.

7. Utilisation d'un amide selon l'une des revendications 1 à 4, pour la préparation d'un agent pharmaceutique pour le traitement de maladies neurodégénératives et de troubles neuronaux.

8. Utilisation selon la revendication 7, pour le traitement de maladies neurodégénératives et de troubles neuronaux, qui sont déclenchés par l'ischémie, un traumatisme ou on des saignements importants.

9. Utilisation selon la revendication 6, pour le traitement d'un accident vasculaire cérébral et d'un traumatisme du cerveau.

10. Utilisation selon la revendication 6, pour le traitement de la maladie d'Alzheimer et de la maladie de Huntington.

11. Utilisation selon la revendication 6, pour le traitement de l'épilepsie.

12. Utilisation d'un amide selon l'une des revendications 1 à 4, pour la préparation d'un agent pharmaceutique pour le traitement de troubles du coeur après ischémies cardiaques, troubles des reins après ischémie rénale, troubles des muscles squelettiques, dystrophies musculaires, troubles qui se forment par prolifération des cellules des muscles lisses, du vasospasme coronaire, du vasospasme cérébral, de la cataracte des yeux et de la resténose des vaisseaux sanguins après angioplastie.

13. Utilisation d'un amide selon l'une des revendications 1 à 4, pour la préparation d'un agent pharmaceutique pour le traitement de tumeurs et de leurs métastases.

14. Utilisation d'un amide selon l'une des revendications 1 à 4, pour la préparation d'un agent pharmaceutique pour le traitement de maladies, dans lesquels des niveaux augmentés en interleukine-1 se présentent.

15. Utilisation d'un amide selon l'une des revendications 1 à 4, pour la préparation d'un agent pharmaceutique pour le traitement de maladies immunologiques comme des inflammations et des maladies rhumatismales.

16. Compositions pharmaceutiques pour administration pérorale, parentérale ou intrapéritonéale, contenant par dose unitaire, en plus des auxiliaires pharmaceutiques usuels, au moins un amide selon l'une des revendications 1 à 4.
